# EUROPEAN PATENT SPECIFICATION

(11) **EP 4 355 261 B1**
(45) Date of publication and mention of the grant of the patent: **29.07.2026**
(21) Application number: 22738205.8
(22) Date of filing: 13.06.2022
(51) Int. Cl.: A61F 2/07, A61F 2/06

(54) **SUPPORT RING FOR VASCULAR AORTIC REPAIR**
STÜTZRING ZUR GEFÄSSAORTENREPARATUR
RÉPARATION AORTIQUE VASCULAIRE À ANNEAU DE SUPPORT

(30) Priority: 14.06.2021 US 202163210258 P
(43) Date of publication of application: 24.04.2024
(73) Proprietor: Bolton Medical, Inc., Sunrise, FL 33325 (US)
(72) Inventor: GARCIA, Eduardo, Alejandro, Sunrise, FL 33325 (US); LOSTETTER, Timothy, Sunrise, FL 33325 (US); MAGEN, Eitan, Sunrise, FL 33325 (US)
(74) Representative: Graham Watt & Co
(86) International application number: PCT/US2022/033247
(87) International publication number: WO 2022/265989

(56) References cited:
- EP-A1- 1 673 040
- EP-A1- 2 749 250
- US-A1- 2015 216 686

## Description

### BACKGROUND OF THE INVENTION

Fenestrated endovascular aortic repair (FEVAR) is a minimally invasive procedure to treat aortic aneurysms that span blood vessels that branch from the aorta that supply blood to vital organs including the kidneys, intestine and liver. Endovascular grafts employed in FEVAR define fenestrations for insertion of branch prostheses that serve as passageways for blood flow through arterial branches to the vital organs. Maximizing blood flow to vital organs and minimizing endoleaks following repair of aneurysms with fenestrated vascular prostheses, such as juxtarenal aortic aneurysms and short-neck abdominal aortic aneurysms, present medical challenges that must be overcome or minimized if additional surgical intervention is to be avoided.

Therefore a need exists for new and improved endovascular repair devices and methods to treat aortic pathologies, in particular in the perivisceral segments of the aorta, such as juxtarenal and short-neck abdominal aortic aneurysms.

EP 1673040 A1 describes a prothesis having a variable size or stretchable fenestration in the graft material of a tubular graft. US2015/216686 A1 describes a kit including a first implant comprising a first tubular body defining a window and a second implant comprising a second tubular body designed to be positioned in the window. EP 2749250 A1 describes an endoluminal prosthesis with one or more fenestrations.

### SUMMARY OF THE INVENTION

The present invention relates to vascular prostheses for use in treating and repairing aortic vascular damage, such as vascular damage associated with aortic aneurysms and regions of the aorta having arterial branches that supply blood to vital organs and tissues, including juxtarenal aortic aneurysms and short-neck abdominal aortic aneurysms.

The invention is defined by the appended claims.

In one embodiment a stent graft includes a tubular graft component that defines an inside surface, an outside surface, an open proximal end, an open distal end, and at least one fenestration. A proximal stent is located at the tubular graft component proximal to the at least one fenestration. A ring is located at the tubular graft component and extends along the tubular graft component and around the at least one fenestration. A liner extends between the inside surface and the outside surface of the tubular graft component, and through the at least one fenestration, the ring thereby being sealed from exposure at one surface of the tubular graft component by the liner, and at the other surface of the tubular graft component by the tubular graft component and the liner. The ring extends along the tubular graft component outside a perimeter that defines the fenestration, whereby some portion of tubular graft component extends from the ring to the perimeter of the fenestration defined by the tubular graft component, such that the diameter of the fenestration, defined by tubular graft component, is smaller than that of the internal diameter of the ring.

Also described is a method for treating an aortic aneurysm, including the step of delivering a stent graft through an artery to an aneurysm of a patient, the aneurysm spanning a region of the artery that includes at least one arterial branch, the stent graft being radially constricted by a stent graft delivery device, the stent graft including: a tubular graft component defining an inside surface, an outside surface, an open proximal end, an open distal end, and at least one fenestration; a proximal stent at the tubular graft component proximal to the at least one fenestration; a ring at the tubular graft component and extending along the tubular graft component and around the at least one fenestration; and a liner extending between the inside surface and the outside surface, and through at least one fenestration, the ring thereby being sealed from exposure at one surface of the tubular graft component by the liner, and at the other surface of the tubular graft component by the tubular graft component and the liner. The at least one fenestration is aligned with the at least one arterial branch at the aneurysm site of the patient. At least one branch prosthesis is delivered through the proximal open end of the distal open end of the tubular graft component of the stent graft, and through the at least one fenestration to the arterial branch, the branch prosthesis being radially and releasably constricted by a branch prosthesis delivery device. The branch prosthesis is released from the branch prosthesis delivery device, resulting in expansion of the branch prosthesis that causes contact at the tubular graft component, thereby forming a seal between the stent graft and the branch prosthesis and treating the aortic aneurysm.

The present invention has several advantages. For example, protection against wear between the ring at the fenestration and a bridging stent extending through the fenestration to a branch blood vessel of the aorta is significantly reduced by sealing the ring from exposure at one surface of a tubular graft component by a liner, and at the other surface of the tubular graft component by the tubular graft component and the liner. Specifically, rather than a single layer of protection between the ring and a bridging stent afforded by the liner extending through a fenestration, the tubular graft provides an additional layer of protection against wear between the ring and the bridging stent. In various embodiments, the ring can be employed as support for the fenestration or, alternatively, can be formed of radiopaque markers that are either linked and affixed to the tubular graft component on the stent graft. In one embodiment, radiopaque markers of the ring are distinct from each other and are separately secured to the tubular graft component, such as by use of fabric that independently encapsulates the radiopaque markers of the ring, thereby providing further protection against wear between the ring, or components of the ring, and a bridging stent extending through the fenestration defined by the tubular graft component. In such embodiments, where the ring is composed of distinct radiopaque markers, an additional ring formed of a suitable material, such as nitinol, can be affixed to the tubular graft component and extend about a perimeter of the fenestration, thereby providing support for the fenestration while also enabling the radiopaque markers of the ring to clearly mark the fenestration during implantation of the stent graft of the invention and a bridging stent graft at a surgical site of a patient.

### BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.
FIG. 1 is a side view of one embodiment of a stent graft of the invention.
FIG. 2 is a cross-section, in part, of an embodiment of the invention shown in FIG. 1, wherein a bridging stent extends through a fenestration defined by the tubular graft component of the stent graft of the invention, showing where an additional layer of protection against wear between the markers and the bridging stent as provided by the presence of both the liner and the wall of the tubular graft component.
FIG. 3 is a perspective view of one embodiment of a liner employed in the stent graft of the invention, wherein two discs, each having a fenestration, are aligned and sewn together at the aligned fenestrations.
FIG. 3A is an exploded view of the liner shown in FIG. 3.
FIG. 4 is a detail of one embodiment of the stent graft of the invention that includes a ring of radiopaque markers about a fenestration of the stent graft of FIG. 1, wherein a liner extending between an inside surface and an outside surface of the tubular graft component of the stent graft is shown in dotted outline.
FIG. 4A is a cross-sectional view of the detail shown in FIG. 4 taken along lines 4A-4A of FIG. 4.
FIG. 5 is a detail of one embodiment of a ring of radiopaque markers about a fenestration of the stent graft shown in FIG. 1, wherein the radiopaque markers are each encapsulated within fabric that are shown in dotted outlines apart from the liner, and wherein a liner of the stent graft extending between the inside surface in the outside surface of the tubular graft component is also shown in dotted outline.
FIG. 5A is a cross-section of the detail shown in FIG. 5 taken along lines 5A-5A of FIG. 5.
FIG. 6 is a detail of one embodiment of the invention shown in FIG. 1, wherein a continuous support ring extends around a perimeter of the liner extending between the inside surface and the outside surface of the tubular graft component of the stent graft.
FIG. 6A is a cross-section of the detail shown in FIG. 6 taken along lines 6A-6A of FIG. 6.
FIG. 7 is a detail of one embodiment of the invention shown in FIG. 1, wherein the ring is a continuous support ring that extends around a fenestration defined by the tubular graft component, and wherein the supporting ring is secured between one side of the tubular graft component of the stent graft and the liner.
FIG 7A is a cross-section of the detail shown in FIG. 7 taken along lines 7A-7A of FIG. 7.
FIG. 8 is a detail of one embodiment of the invention shown in FIG. 1, wherein radiopaque markers at one surface of the tubular graft component are between the support ring and the fenestration of the tubular graft component.
FIG. 8A is a cross-section of the detail shown in FIG. 8 taken along lines 8A-8A of FIG. 8.
FIG. 9 is a detail of one embodiment of the invention shown in FIG. 1. Wherein the ring is a continuous supporting ring and wherein a radiopaque marker that is a continuous ring between the support ring and the fenestration of the tubular graft component.
FIG. 9A is a cross-section of the detail shown in FIG. 9 taken along lines 9A-9A of FIG. 9.
FIG. 10 is a detail of one embodiment of the invention shown in FIG. 1, wherein the ring is a support ring between a surface of the tubular graft component and the liner, and further including a plurality of radiopaque markers.
FIG. 10A is a cross-section of the detail shown in FIG. 10 taken along lines 10A-10A of FIG. 10.
FIG. 11 is a side view of another embodiment of a stent graft of the invention, including a stent proximal to a fenestration of a tubular graft component that is a bare stent.
FIG. 11A is a detail of the embodiment of the invention shown in FIG. 11 showing a proximal apex and proximal barb of the bare stent of FIG. 11.
FIG. 12 is a side view of an embodiment of the stent graft of the invention, wherein stents distal to the fenestration of the tubular graft component are at the proximal end of an extender stent graft placed within the legs of the bifurcated tubular graft component.
FIG. 13 is a side view of still another embodiment of the stent graft of the invention, wherein stents are immediately proximal and distal to the fenestration defined by the tubular component.
FIG. 14 is a side view of yet another embodiment of the stent graft of the invention, wherein the stent graft includes stents immediately proximal and distal to the fenestration, as well as a bare stent at a proximal end of the tubular graft component, and stents at the proximal end of an extender stent graft placed within the legs of the bifurcated tubular component.
FIG. 15 is a side view of a stent graft of the invention, wherein branch prostheses have been implanted through respective fenestrations and associated fenestration rings of the stent graft of the invention.
FIG. 16 is a side view of an embodiment of the support ring suitable for use as a component of one embodiment of the invention, showing a wire first end and a wire second end secured to each other by a crimped connector, and wherein at least one length L of the wire between the wire first end and the wire second end traverses a space defined by a helical coil first end and a helical coil second end that are in opposition to each other on either side of the crimped connector.
FIG. 16A is a view of the detail of the support ring of FIG. 16, taken along line 16A-16A.
FIG. 16B is a detail of the support ring of FIG. 16, showing the crimped connector having been crimped at two points to thereby secure the wire first end and the wire second end together.
FIG. 16C is a side view of a support wire that has been straightened to show the length L between the first end and the second end of the wire that traverses the distance between the first end and the second end of the helical coil of FIG. 16 and 16A.
FIG. 17 is a side view of another embodiment of a branch sleeve, having a support ring fixed to an outside surface of the branch sleeve, wherein the branch sleeve is fixed to a liner covering a sealing ring at a fenestration of a tubular graft, and a branch stent graft secured at the branch sleeve by an interference fit with the branch sleeve.

### DETAILED DESCRIPTION OF THE INVENTION

The invention is generally directed to vascular prostheses for use in treating and repairing vascular damage, such as vascular damage associated with an aortic aneurysm at regions of the aorta having arterial branches that supply blood to vital organs and tissues.

A description of example embodiments follows.

When reference is made to a prosthesis, also referred to herein as "stent graft," "stent graft prosthesis," or "vascular prosthesis," to be delivered, or implanted in a patient, the word "proximal" means that portion of the prosthesis or component of the prosthesis that is relatively close to the source of blood flow from the heart of the patient. "Distal" means that portion of the prosthesis or component of the prosthesis that is relatively far from the source of blood flow from the heart of the patient. "Cranial," as that term is used herein, means relatively close in absolute distance to the heart of the patient. "Caudal," as that term is used herein, means relatively far in absolute distance to the heart of the patient. It is to be understood that, therefore, a proximal end of a prosthesis can be cranial or caudal to the heart of a patient relative to a distal end of the same prosthesis.

When, however, reference is made to a delivery system or component of a delivery system employed to deliver, or implant, a prosthesis, the word, "proximal," as employed herein, means closer to the clinician using the delivery system. When reference is made to a delivery system or component of a delivery system, "distal," as that term is employed herein, means, further away from the clinician using the delivery system.

For clarity, the word "proximate" means "close to," as opposed to the meanings ascribed to "proximal" or "distal" described above with respect to either the prosthesis or a delivery system.

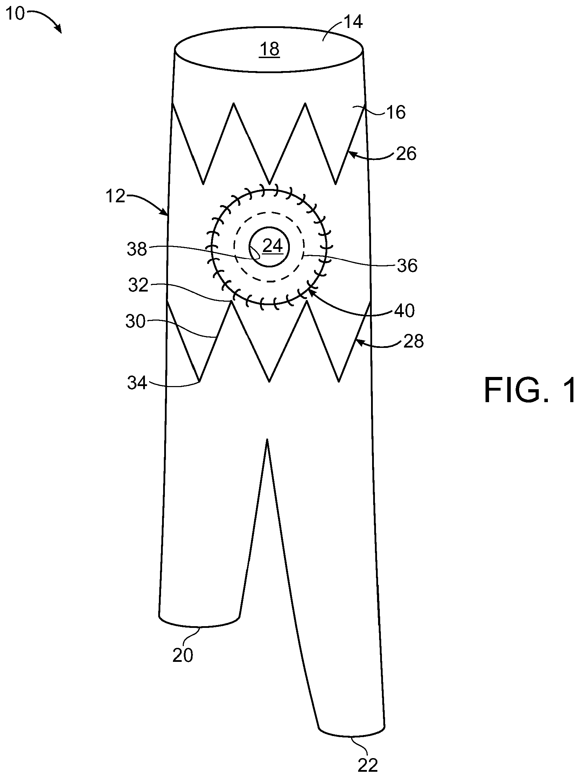

One embodiment of the stent graft of the invention is shown in FIG. 1. As shown therein, stent graft 10 includes tubular graft component 12 that defines inside surface 14, outside surface 16, open proximal end 18, and an open distal end. The open distal end, as shown in FIG. 1 can be bifurcated, thereby defining first open distal end 20 and second open distal end 22. Tubular graft component 12 further defines at least one fenestration 24. Proximal stent 26 extends about a circumference of tubular graft component 12 proximal to fenestration 24. Distal stent 28 extends about the circumference of tubular graft component 12 distal to the at least one fenestration 24. In embodiments, the stent graft includes one, two, three, four, or five fenestrations and one, two, three, four, or five rings around the one, two, three, four, or five fenestrations, respectively.

Proximal stent 26 and distal stent 28, as shown in FIG. 1, include struts 30 that join at opposite ends to define proximal apices 32 and distal apices 34. Proximal stent 26 and distal stent 28 are, at least in one embodiment, radially self-expanding. Typically, proximal stent 26 and distal stent 28 are formed of a shape-memory alloy, such as nitinol. In other embodiments, stent graft 10 includes additional stents distributed along a longitudinal length of tubular graft component 12. Also, in specific embodiments, fenestration 24 can be nested between struts 30 of proximal stent 26 or distal stent 28, or both.

Tubular graft component 12 is fabricated of a suitable material, such as is known in the art. Examples of suitable materials of tubular graft include polytetrafluoroethylene (PTFE), such as ePTFE, and polyethylene terephthalate (PET), such as woven polyester.

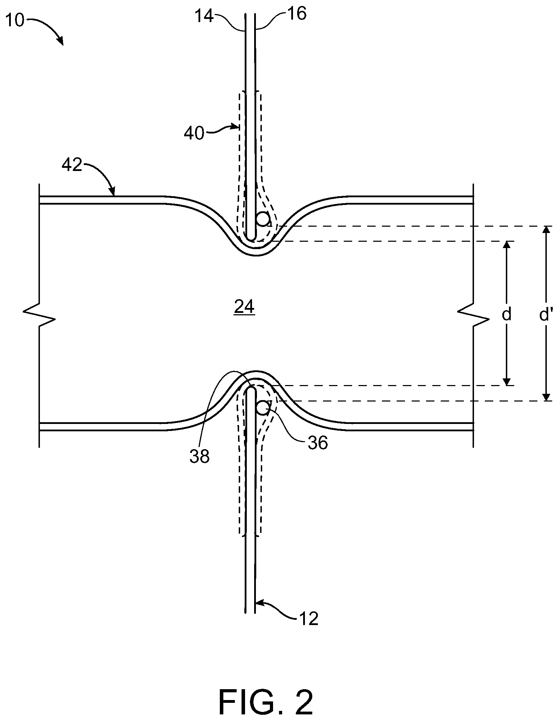

Ring 36 is at tubular graft component 12 and extends along tubular graft component 12 and around fenestration 24. In the embodiment shown in FIG. 1, ring 36 extends along tubular graft component 12 outside perimeter 38 that defines fenestration 24, whereby some portion of tubular graft component 12 extends from ring 36 to perimeter 38 of fenestration 24 defined by tubular graft component 12. As can be seen in FIG. 2, which is a cross-section, in part, of tubular graft component 12 of stent graft 10 shown in FIG. 1, diameter ***d*** of fenestration 24, defined by tubular graft component 12, is smaller than that of the internal diameter ***d***' of ring 36.

Liner 40 extends between the inside surface 14 and outside surface 16 of tubular graft component 12, and through fenestration 24, thereby sealing ring 36 from exposure at one surface of tubular graft component 12 by liner 40, and at the other surface 14 of tubular graft component 12 by tubular graft component 12 and liner 40. Ring 36, shown in FIG. 2, cannot be seen in FIG. 1 because it is sealed by liner 40 from exposure.

As can be seen from FIG. 2, where bridging stent graft 42 extends through fenestration 24 of stent graft component 12, contact between bridging stent graft 42 (also referred to herein as "branch stent graft" or "branch prosthesis") and ring 36 is prevented on one side 16 of tubular graft component 12 by liner 40 and on the other side 14 of tubular graft component 12 by the material of tubular graft component 12 and liner 40. The additional layer of protection afforded by the additional layer of tubular graft component 12 between ring 36 and bridging stent graft 42 reduces the likelihood of wear of fabric between ring 36 and bridging stent graft 42 and consequent contact between ring 36 and bridging stent graft 42. Bridging stent graft 42 can be fabricated of at least one of stainless steel and cobalt chromium. Ring 36 can be fabricated of a metal or metal alloy, such as nitinol. Reduced wear of fabric between ring 36 and bridging stent graft 42 diminishes loss of the integrity of the fabric of liner 40 over time, thereby reducing the likelihood of leakage of blood across a seal formed between ring 36 and bridging stent graft 42.

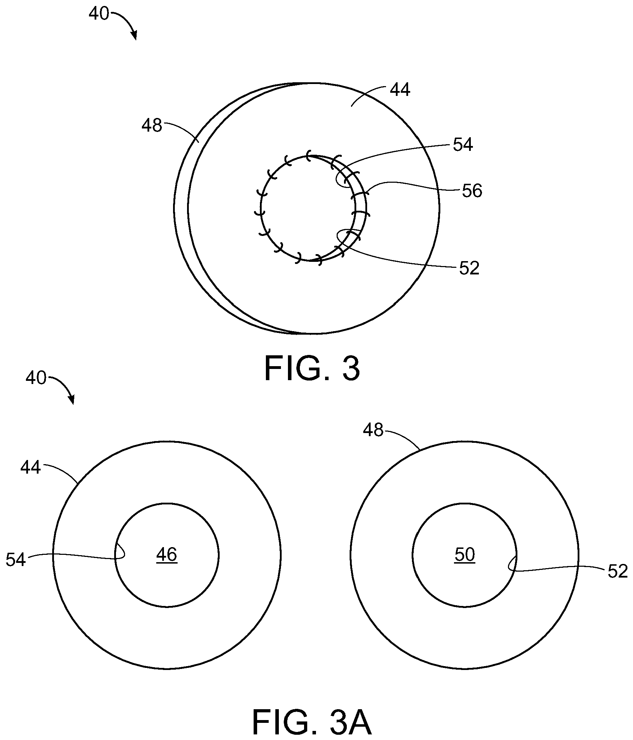

FIG. 3 is a perspective view of one embodiment of liner 40 shown in FIGs. 1 and 2, but without the presence of tubular graft component 12 in order to clarify the construction of at least one embodiment of liner 40. As shown in FIG. 3, liner 40 includes first layer 44 defining first opening 46, and second layer 48 defining second opening 50. First opening 46 and second opening 50 are aligned and secured to each other at perimeters 52,54 of first opening 46 and second opening 50, respectively, such as by use of sutures 56. FIG 3A is an exploded view of liner 40 of FIG. 3, showing first layer 44 and second layer 48 separately, and first opening 46 and second opening 50 separately. Alternatively, first layer 44 and second layer 48 can be secured to each other at openings 46,50 by other suitable means known in the art, such as by use of an adhesive. In an alternative embodiment, not shown, liner 40 can be fabricated of a single component that has been woven or molded, for example, to conform to perimeter 38 of fenestration 24 and to be fixed at inside surface 14 and outside surface 16, such as is shown in FIGs. 1 and 2. Liner 40 need not be fabricated of a woven fabric, but can, instead, and in certain embodiments, be a continuous material, formed by molding a suitable polymer in an appropriate shape. Examples of suitable materials include, for example, polytetrafluoroethylene (PTFE), such as ePTFE, and polyethylene terephthalate (PET).

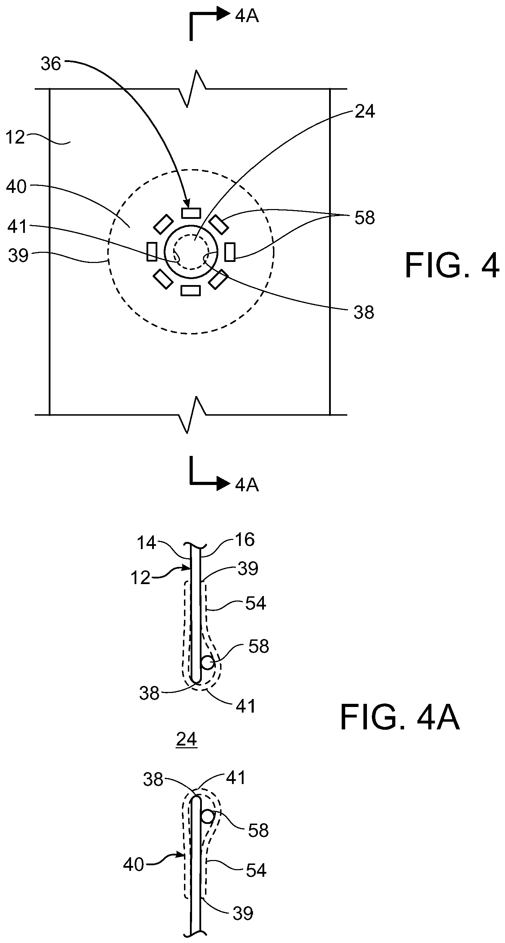

FIG. 4 is a detail of one embodiment of stent graft 10 of FIG. 1, wherein ring 36 is a circular arrangement of markers 58 distributed about perimeter 38 of fenestration 24 of tubular graft component 12. As illustrated in FIG. 4, edges 39,41 of liner 40 are represented as dotted lines in order to show the spatial relationship between radiopaque markers 58 of ring 36 and fenestration 24.

FIG. 4A is a side view of the detail shown in FIG. 4 taken along line 4A-4A of FIG. 4. As can be seen in FIG. 4A, liner 40 extends through fenestration 24, thereby sealing radiopaque markers 58 from exposure at one surface 16 of tubular graft component 12 by liner 40, and at the other surface 14 of tubular graft component 12 by tubular graft component 12 and liner 40. Radiopaque markers 58 of ring 36 are secured to tubular graft component 12 by a suitable means, such as is known in the art, including, for example, sutures, adhesive, etc. Liner 40 is secured at perimeter 54 at inside surface 14 and outside surface 16 by suitable means.

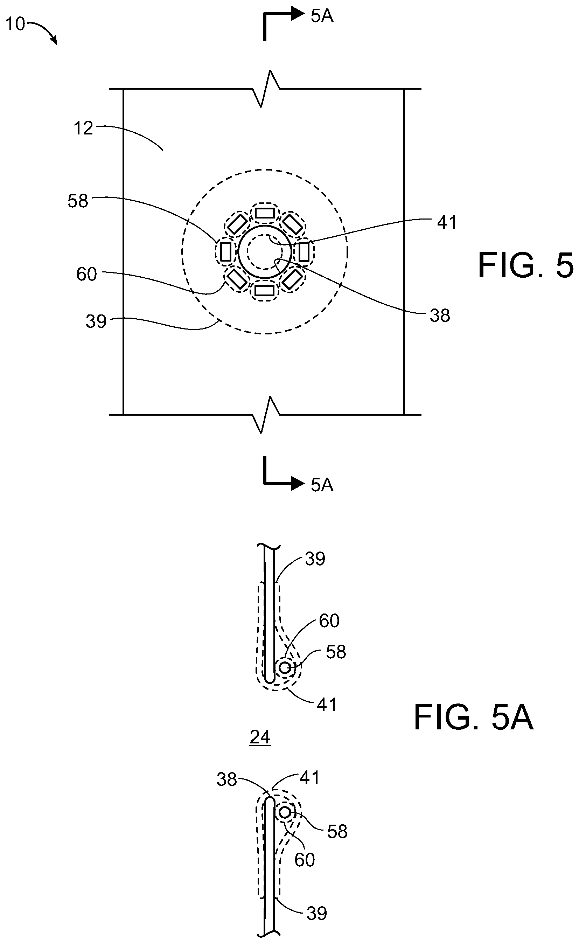

FIG. 5 is a detail of another embodiment of stent graft 10 shown in FIG. 1, wherein radiopaque markers 58 are each encapsulated by fabric 60, and wherein the fabric 60 encapsulating each radiopaque marker 58 is secured to tubular graft component 12 by sutures or an adhesive.

FIG. 5A is a cross-sectional view of the detail shown in FIG. 5, taken along lines 5A-5A of FIG. 5, also illustrating encapsulation of radiopaque markers 58 by fabric 60, wherein the fabric 60 encapsulating each radiopaque marker 58 is itself sealed from exposure by liner 40.

It is to be understood in radiopaque markers can have alternative shapes, such as an elongated shape, as shown in FIGs. 5 and 5A. Alternatively, radiopaque markers can be circular, asymmetric D-shaped, spherical, or a continuous ring.

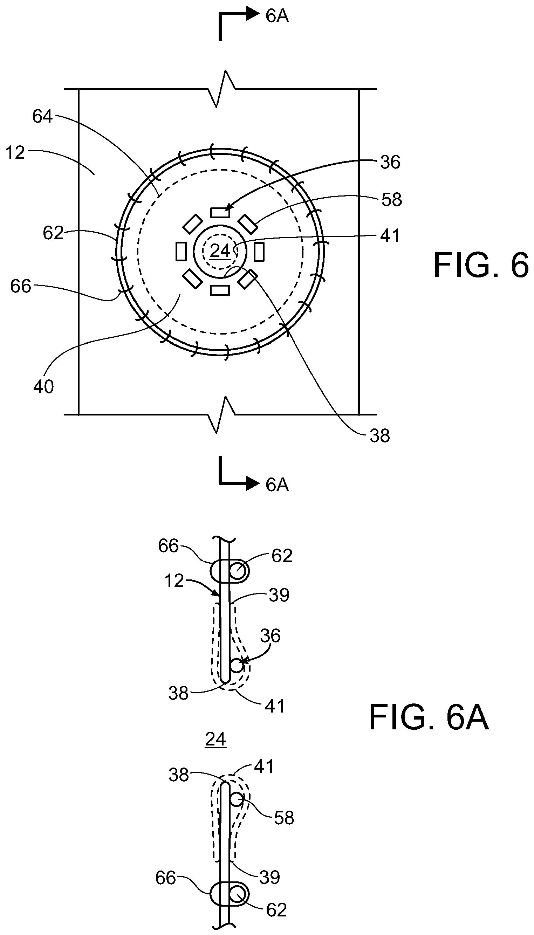

FIG. 6 is a detail of another embodiment of a stent graft of the invention, wherein support ring 62 extends about outside perimeter 64 of liner 40. Ring 36 of the invention is a plurality of separate radiopaque markers 58, such as was shown in FIGs. 4 and 4A.

FIG. 6A is a cross-section of the embodiment shown in FIG. 6 taken along line 6A-6A, and shows support ring 62 secured to tubular graft component 12 by sutures 66.

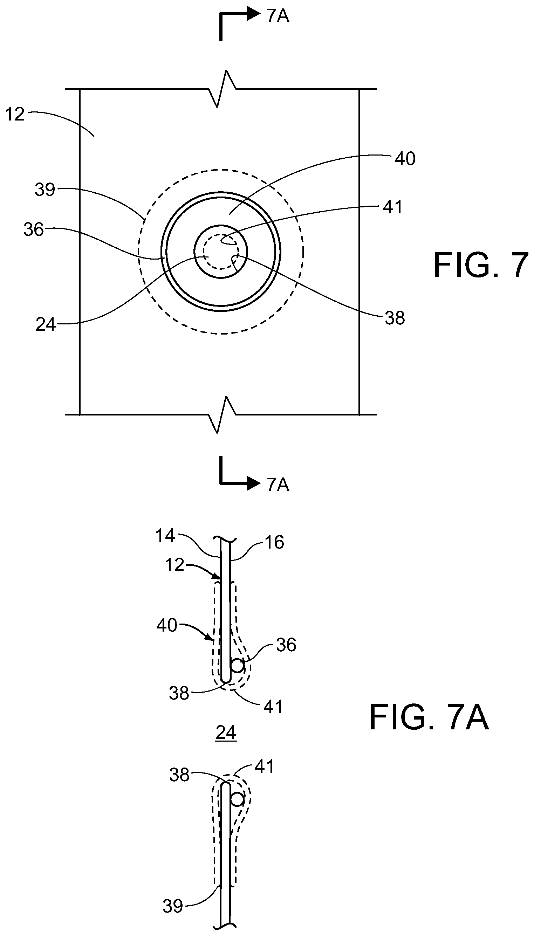

FIG. 7 is a detail of one embodiment of the invention shown in FIG. 1, wherein ring 36 is a continuous support ring that extends around fenestration 24 defined by tubular graft component 12, and wherein support ring 36 is secured between one side 16 of the tubular graft component 12 of stent graft 10 and liner 40. Liner 40 has perimeter 39.

FIG. 7A is a cross-section of the detail shown in FIG. 7 taken along lines 7A-7A of FIG. 7, showing ring 36 sealed within liner 40.

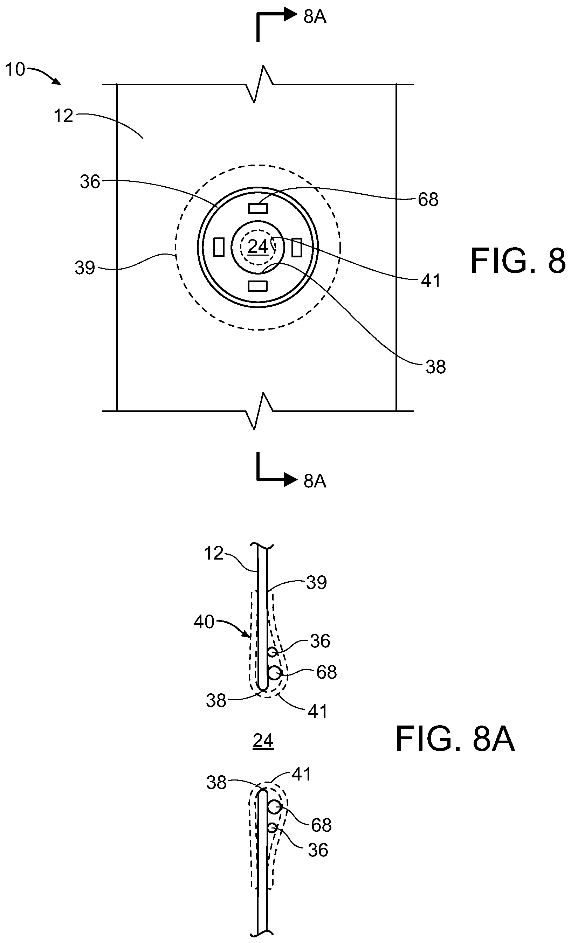

FIG. 8 is a detail of one embodiment of the invention shown in FIG. 1, wherein radiopaque markers 68 at one surface of tubular graft component 12 are between support ring 36 and fenestration 24 of tubular graft component 12.

FIG. 8A is a cross-section of the detail shown in FIG. 8 taken along lines 8A-8A of FIG. 8, showing that both support ring 36 and radiopaque markers 68 are sealed within liner 40.

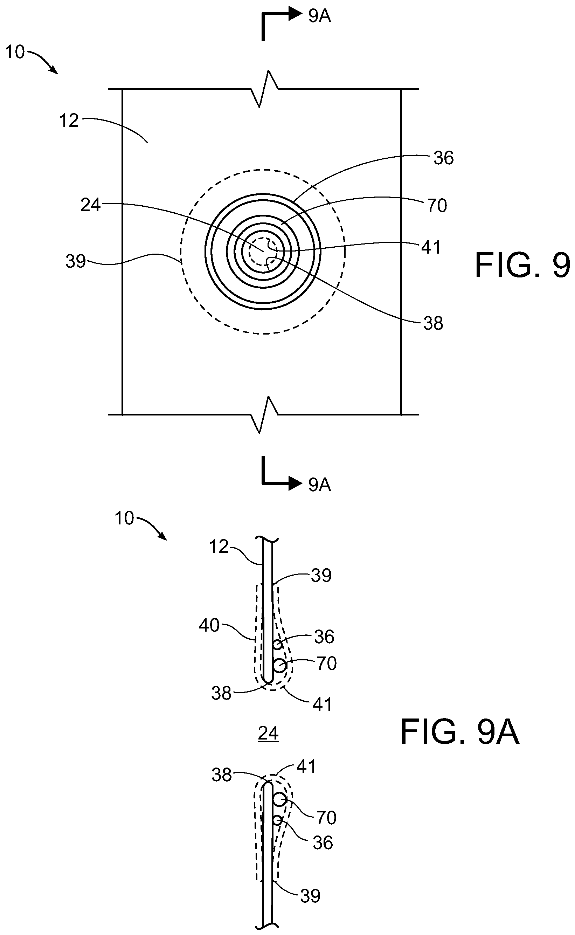

FIG. 9 is a detail of one embodiment of the invention shown in FIG. 1, wherein ring 36 is a continuous support ring and wherein a radiopaque marker 70 also is a continuous ring and is between support ring 36 and fenestration 24 of tubular graft component 12.

FIG. 9A is a cross-section of the detail shown in FIG. 9 taken along lines 9A-9A of FIG. 9 and showing both support ring 36 and radiopaque ring 70 to be sealed within liner 40.

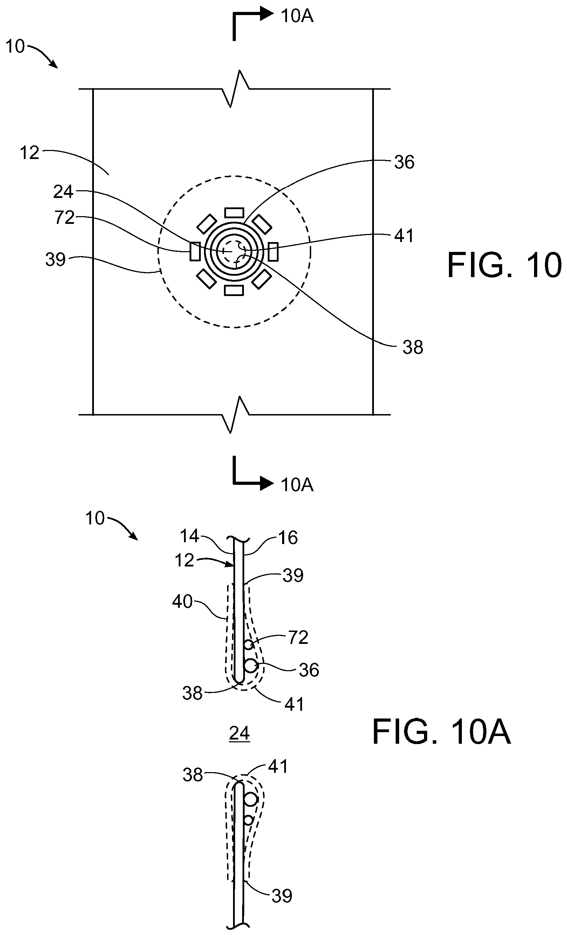

FIG. 10 is a detail of one embodiment of the invention shown in FIG. 1, wherein ring 36 is a support ring that is between radiopaque markers 72 extending around ring 36 and fenestration 24.

FIG. 10A is a cross-section of the detail shown in FIG. 10, showing both support ring 36 and radiopaque markers 72 to be between one side 16 of tubular graft component 12 and liner 40.

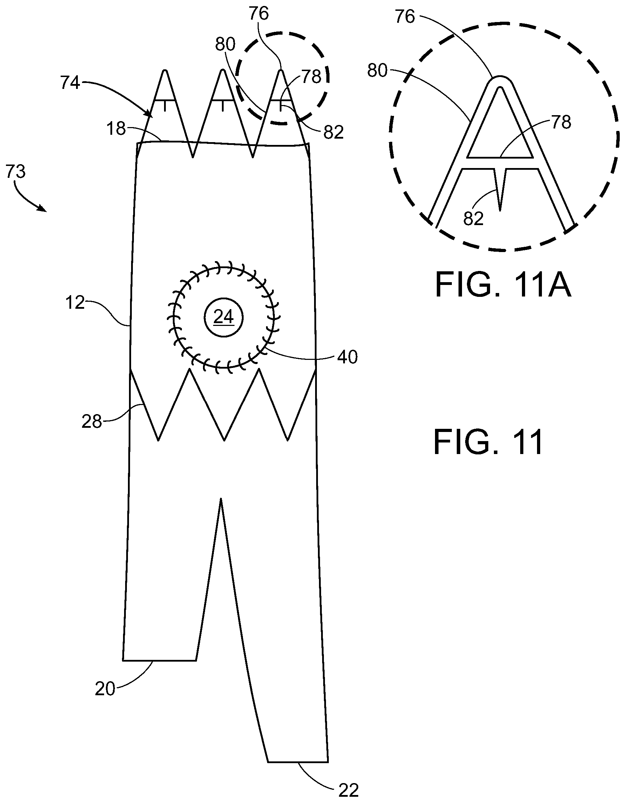

FIG. 11 is a side view of another embodiment of stent graft 73 of the invention, including bare stent 74 proximal to fenestration 24 of tubular graft component 12.

FIG. 11A is a detail of the embodiment of stent graft 73 of the invention shown in FIG. 11 showing proximal apex 76, wherein bridge 78 extends between struts 80, and proximal barb 82 extends distally from bridge 78 of bare stent 74.

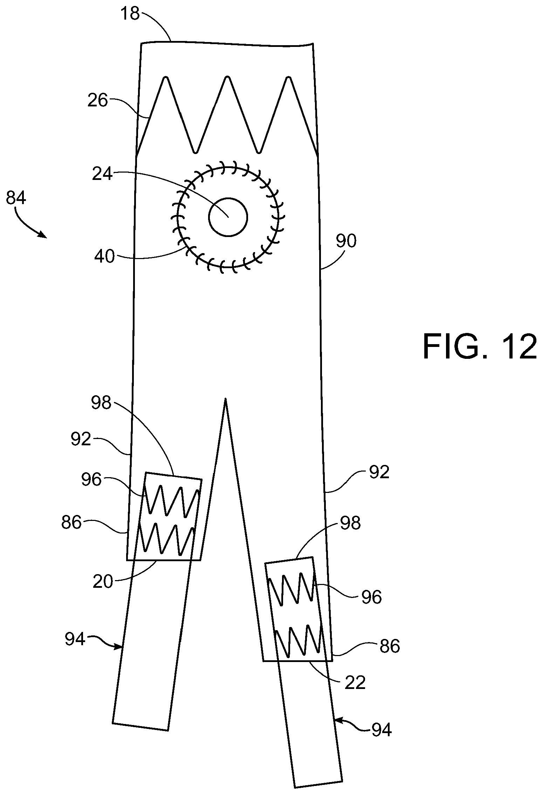

FIG. 12 is a side view of an embodiment of stent graft 84 of the invention wherein stents 86 distal to fenestration 24 of tubular graft component 90 are at the proximal end of an extender stent graft placed in the legs 92 of the bifurcated tubular graft component. As can be seen therein, prosthetic extensions 94 (also referred to herein as "extender stent grafts") extend distally from the distal end 86 of each leg 92, and each prosthetic extension 94 includes a stent 96 at its proximal end 98.

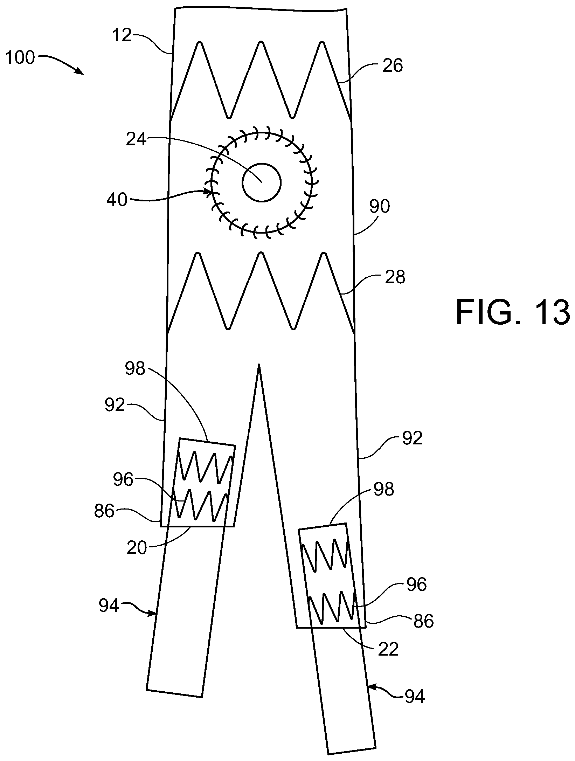

FIG. 13 is a side view of still another embodiment wherein stent graft 100 of the invention includes struts 26, 28 that are immediately proximal and distal to fenestration 24, defined by tubular graft component 12. Stents 96 are at proximal ends 98 of an extender stent grafts 94 placed within legs 92 of tubular graft component 12.

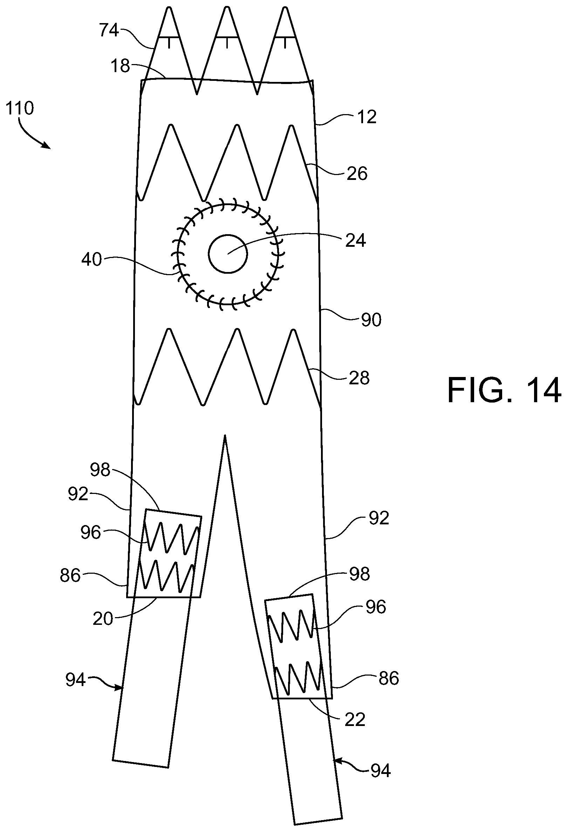

FIG. 14 is a side view of yet another embodiment of the invention wherein stent graft 110 includes stents 26, 28 immediately proximal and distal to fenestration 24, as well as bare stent 74 at a proximal end 18 of tubular graft component 12 and stents 96 of proximal end 98 of extender stent graft 94 placed within legs 92 of bifurcated tubular graft component 12.

Stent grafts can be implanted at a surgical site that spans an aneurysm, in particular in the perivisceral segment of the aorta, by suitable methods, such as is known in the art. Following implantation, bridging stents can be delivered through the fenestration, fenestration ring and into a branch of the aorta, such as renal, superior mesenteric or celiac arteries. Suitable delivery devices for implanting stent grafts are described, for example, in U.S. Patent Application Nos: 63/111,357, 63,153,701, and 63,210,381.

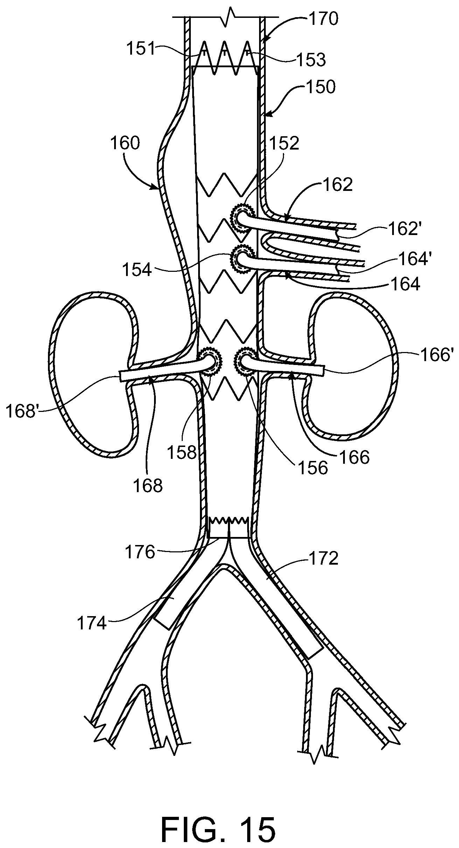

In an embodiment, as shown in FIG. 15, stent graft 150 that includes fenestration rings 152,154,156,158 is implanted at aneurysm site 160. Branch prostheses 162,164,166,168 are delivered through each corresponding fenestration and extend from each corresponding fenestration. Distal ends 162',164',166',168' are each directed by a respective branch prosthesis delivery device (not shown) into a respective branch of aorta 170 at aneurysm 160 and secured by fenestration rings 152,154,156,158 in respective fenestrations at proximal ends and within branch artery at distal ends. Proximal ends of respective branch prostheses 162,164,166,168 are fixed at respective fenestrations by fenestration rings 152,154,156,158. Each branch prosthesis 162,164,166,168 is then released from the respective branch prosthesis delivery device. The vascular prosthesis delivery device and the branch prosthesis delivery device are then removed either simultaneously, or in sequence, thereby completing implantation and treatment of the aneurysm. In an embodiment, additional branch prostheses 172,174 can be implanted into distal end 176 of stent graft 150. Bare stent 151 of stent graft 150 can include barbs 153.

Although not shown, the distal end of the vascular repair device of the invention can be bifurcated and additional prostheses can be implanted into the distal ends of the bifurcated vascular prosthesis.

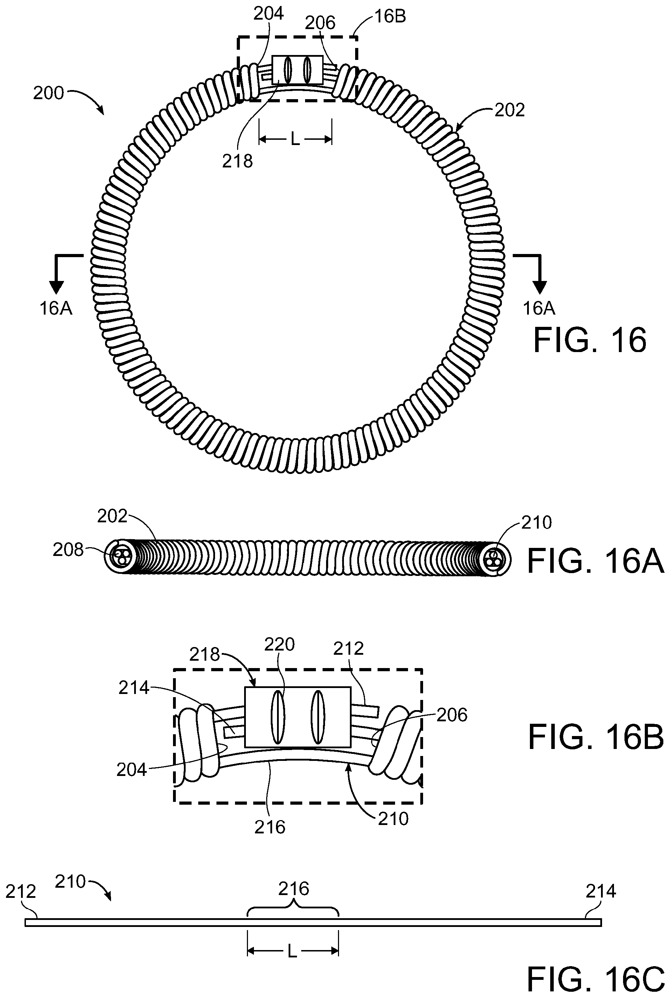

In one particular embodiment, sealing ring 36, shown in FIG. 1, is support ring 200, shown in FIG. 16, and includes helical coil 202 having helical coil first end 204 and helical coil second end 206. As can be seen in FIG. 16A, which is a view of support ring 200 of FIG. 16 taken along line 16A-16A, helical coil 202 defines lumen 208. Referring back to FIG. 16, helical coil 202 extends in an arc, wherein helical coil first end 204 and helical coil second end 206 are in an opposing relation to each other that defines length L that is between helical coil first end 204 and helical coil second end 206 and is outside lumen 208. Wire 210 extends through lumen 208 and, as shown in FIG. 16B, includes wire first end 212 and wire second end 214. As also shown FIG. 16B, wire 210 traverses length **L** of space between helical coil first end 204 and helical coil second end 206 along at least one length 216 of wire 210 between wire first end 212 and wire second end 214. Length 216 of wire 210 between wire first end 212 and wire second end 214 is the length **L** of space between helical coil first end 204 and helical coil second end 206, as shown in FIG. 16C. There are as many lengths 216 of wire 210 between wire first end 212 and wire second end 214 as there are wraps of wire 210 between wire first end 212 and wire second 214, not including the portions of wire 210 that are secured to each other at wire first end 212 and wire second end 214. Wire first end 212 and wire second end 214 are secured to each other by a suitable means, such as by connector 218 that is crimped, as shown in FIG. 16B, which is a detail of support ring 200 of FIG. 16. Connector 218 has two crimps 220, as shown in FIG. 16B, although other numbers of crimps 220 can be employed, such as 1, 3, 4, or 5 crimps 220. Further description of the support ring 200 of FIG. 16 can be found in U.S.S.N. 63/210,265.

In one embodiment, helical coil 202 of support ring 200 is radiopaque. As shown in FIGs. 16, 16A, 16B, and 16C, wire 210 includes two loops, whereby a portion of wire 210 includes portion 216 that traverses length L between helical coil first end 204 and helical coil second end 206. FIG. 16C shows portion 216 of wire 210 that spans length L between helical coil first end 204 and helical coil second end 206. Portion 216 of wire 210 is between wire 210 first end 212 and wire 210 second end 214. In various embodiments, wire 210 can traverse the space having length **L** between helical coil first end 204 and helical coil second end 206 at 2, 3, 4, 5, 6, 7, 8, 9, or 10 portions 216 of length **L** along wire 210 and between wire 210 first end 212 and wire 210 second end 214.

The support ring 200 may be formed by a method including directing wire 210 through lumen 208 defined by helical coil 202 extending in an arc from helical coil first end 204 through helical coil second end 206 in opposition to the helical coil first end 204, and across length **L** of the space defined by helical coil first end 204 and helical coil second end 206 that is outside lumen 208 defined by helical coil 202, whereby wire 210 traverses length **L** of the space between helical coil first end 204 and helical coil second end 206 along at least one length 216 of wire 210 between wire first end 212 and wire second end 214, and securing wire first end 212 to wire second end 214, thereby forming support ring 200, as represented, for example, above, and as described above, with reference to FIGs. 16, 16A, 16B, and 16C. The method may further include the step of fixing the support ring 200 to tubular graft component 12 at fenestration, wherein fenestration 24 is within the arc of helical coil 202, thereby forming another embodiment of an aortic prosthesis of the invention.

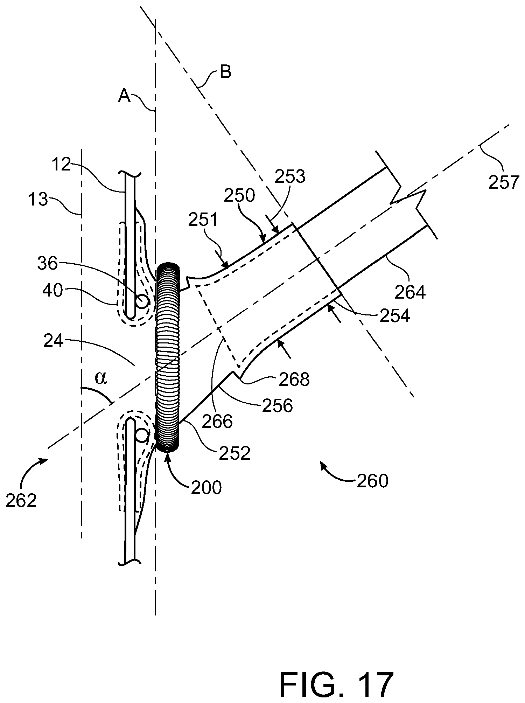

Another example includes graft sleeve 250, such as is shown in FIG. 17, at sealing ring 36 and liner 40 extending around and through, respectively, fenestration 24 of tubular graft component 12. As shown in FIG. 17, graft sleeve 250 includes graft sleeve proximal end 252, graft sleeve distal end 254, and graft sleeve wall 256 extending between the graft sleeve proximal end 252 and the graft sleeve distal end 254. Graft sleeve proximal end 252 has a base diameter and graft sleeve distal end 254 has a diameter less than that of the base diameter of graft sleeve proximal end 252, wherein the graft sleeve proximal end 252 defines plane **A** that intersects plane **B** defined by graft sleeve distal end 254. In one embodiment, graft sleeve 256 defines longitudinal axis 257 that intersects longitudinal axis 13 of tubular graft component 12 at angle **α**. Angle **α** of intersection can range from 0° to 180°, such as an angle of 30°, 60°, 90°, 120°, or 150°. Support ring 200 is fixed to the graft sleeve wall 256 closer to graft sleeve proximal end 252 than to the graft sleeve distal end 254, thereby forming graft sleeve assembly 260. Resulting graft sleeve assembly 260 is fixed to tubular graft wall 12 around fenestration 24, thereby forming aortic prosthesis 262. Branch stent graft 264 is directed through fenestration 24 and is implanted at graft sleeve 250, whereby proximal end 266 of graft sleeve 266 is fixed by interfering relation with graft sleeve 250, as indicated by ridge 268. In one embodiment, graft sleeve is conical, whereby orthogonally transverse first section 251 at a relatively proximal end of graft sleeve 250 is wider than orthogonally transverse second section 253 that is distal to the orthogonally transverse first section 251. The proximal end of the branch stent graft may be flared. Graft sleeve may be conically shaped, and the proximal end of the branch stent may be flared. Further description can be found in U.S.S.N. 63/210,271.

Vascular prostheses of the invention can be implanted, for example, by transfemoral access. Additional branch prostheses that are directed into the vascular prostheses of the invention can be implanted, for example, by supra-aortic vessel access (e.g., through the brachial artery), or by transfemoral access, or access from some other branch or branch of major blood vessels, including peripheral blood vessels.

Also envisaged is a method for treating an aortic aneurysm, comprising the steps of: (a) delivering a stent graft through an artery to an aneurysm of a patient, the aneurysm spanning a region of the artery that includes at least one arterial branch, the stent graft being radially constricted by a stent graft delivery device, the stent graft including: (i) a tubular graft component defining an inside surface, an outside surface, an open proximal end, an open distal end, and at least one fenestration; (ii) a proximal stent at the tubular graft component proximal to the at least one fenestration; (iii) a ring at the tubular graft component and extending along the tubular graft component and around the at least one fenestration; and (iv) a liner extending between the inside surface and the outside surface, and through the at least one fenestration, the ring thereby being sealed from exposure at one surface of the tubular graft component by the liner, and at the other surface of the tubular graft component by the tubular graft component and the liner; (b) aligning the at least one fenestration with the at least one arterial branch at the aneurysm site of the patient; (c) delivering at least one branch prosthesis through the proximal open end or the distal open end of the tubular graft component of the stent graft, and through the at least one fenestration to the arterial branch, the branch prosthesis being radially and releasably constricted by a branch prosthesis delivery device; and (d) releasing the branch prosthesis from the branch prosthesis delivery device, expansion of the branch prosthesis causing contact at the tubular graft component, thereby forming a seal between the stent graft and the branch prosthesis and treating the aortic aneurysm.

There are relevant teachings in U.S. Patent Nos. US 10,987,235, US 11,000,359, US 11,291, 572, US 11,278,390, US 11,219,540, and US 11,154,392; US Published Patent Application Nos.: US 2019/0269498 A1, US 2019/0231514 A1, US 2019/0231571 A1, US 2019/0247178 A1, US 2019/0269497 A1, US 2019/0282355 A1, US 2019/0321207 A1, US 2020/352700A1, and US 2021/0401602 A1; and U.S. Application Serial No: 17/522,251. There are also relevant teachings in United States patent application, titled "Support Ring, Aortic Prosthesis and Method of Forming," filed June 13, 2022, by Eitan Magen and Eduardo Alejandro Garcia.

## Claims

1. A stent graft, comprising:
a) a tubular graft component (12) defining an inside surface (14), an outside surface (16), an open proximal end (18), an open distal end, and at least one fenestration (24);
b) a proximal stent at the tubular graft component proximal to the at least one fenestration;
c) a ring (36) at the tubular graft component (12) and extending along the tubular graft component (12) and around the at least one fenestration (24); and **characterized by**:
d) a liner (40) extending between the inside surface (14) and the outside surface (16), and through the fenestration(24), the ring thereby being sealed from exposure at one surface (16) of the tubular graft component by the liner (40), and at the other surface of the tubular graft component by the tubular graft component (12) and the liner (40); and in that:
the ring (36) extends along the tubular graft component (12) outside a perimeter (38) that defines the fenestration (24), whereby some portion of tubular graft component (12) extends from the ring (36) to the perimeter (38) of the fenestration (24) defined by the tubular graft component (12), such that the diameter of the fenestration (24), defined by tubular graft component (12), is smaller than that of the internal diameter of the ring (36).

2. The stent graft of claim 1, wherein the liner (40) includes a first layer (44) defining a first opening (46), and a second layer (48) defining a second opening (50), wherein the first opening (46) and the second opening (50) are aligned and the first layer (44) and the second layer (48) are secured to each other at the first opening (46) and the second opening (50), and wherein the first layer (44) and the second layer (48) each define a perimeter at which they each are secured to one of the inside surface or the outside surface of the tubular graft component (12).

3. The stent graft of claim 2, wherein the ring (36) includes a plurality of radiopaque marker bands.

4. The stent graft of claim 3, wherein at least a portion of the radiopaque marker bands are encapsulated by fabric (60) apart from the liner (40), and optionally the fabric (60) apart from the liner (40) that encapsulates the radiopaque markers is fixed to the tubular graft component (12).

5. The stent graft of claim 3, wherein the radiopaque marker bands are sewn to the tubular graft component (12).

6. The stent graft of claim 3, wherein at least a portion of the radiopaque markers (58) are shaped as at least one of elongate cylinders and tubes.

7. The stent graft of claim 3, further including a support ring (62) extending around the fenestration and optionally one or more of: is self-expanding, includes shape-memory alloy, and is sealed by the liner (40).

8. The stent graft of claim 7, wherein the support ring (62) extends around the perimeter of the first layer or the second layer.

9. The stent graft of claim 7, wherein the support ring (62) is sewn to the tubular graft component (12).

10. The stent graft of claim 1, wherein the ring (36) is a continuous support ring.

11. The stent graft of claim 10, further including at least one radiopaque marker (68, 70, 72) between the tubular graft component (12) and the liner (40).

12. The stent graft of claim 11, wherein the at least one radiopaque marker (68, 70) extends around the fenestration (24) of the tubular graft component (12), or between the continuous support ring (36) and the fenestration (24) of the tubular graft component (12).

13. The stent graft of claim 12, wherein the at least one radiopaque marker (72) extends around the continuous support ring, whereby the continuous support ring (36) is between the radiopaque marker and the fenestration (24) of the tubular graft component (12).

14. The stent graft of claim 1, wherein the tubular graft component (12) is bifurcated distal to the fenestration (24).

## Patentansprüche

1. Stentgraft, umfassend:
a) eine röhrenförmige Graftkomponente (12), die eine innere Oberfläche (14), eine äußere Oberfläche (16), ein offenes proximales Ende (18), ein offenes distales Ende und mindestens eine Fenestration (24) definiert;
b) einen proximalen Stent an der röhrenförmigen Graftkomponente proximal zu der mindestens einen Fenestration;
c) einen Ring (36) an der röhrenförmigen Graftkomponente (12) und der sich entlang der röhrenförmigen Graftkomponente (12) und um die mindestens eine Fenestration (24) herum erstreckt; und **gekennzeichnet durch:**
d) eine Auskleidung (40), die sich zwischen der inneren Oberfläche (14) und der äußeren Oberfläche (16) und durch die Fenestration (24) hindurch erstreckt, wobei dadurch der Ring an einer Oberfläche (16) der röhrenförmigen Graftkomponente durch die Auskleidung (40) und an der anderen Oberfläche der röhrenförmigen Graftkomponente durch die röhrenförmige Graftkomponente (12) und die Auskleidung (40) gegen Freilegung abgedichtet wird; und **dadurch, dass:**
sich der Ring (36) entlang der röhrenförmigen Graftkomponente (12) außerhalb eines Umfangs (38) erstreckt, der die Fenestration (24) definiert, wobei sich ein gewisser Teil der röhrenförmigen Graftkomponente (12) von dem Ring (36) zu dem Umfang (38) der Fenestration (24), die durch die röhrenförmige Graftkomponente (12) definiert ist, erstreckt, sodass der Durchmesser der Fenestration (24), der durch die röhrenförmige Graftkomponente (12) definiert ist, kleiner als der Innendurchmesser des Rings (36) ist.

2. Stentgraft nach Anspruch 1, wobei die Auskleidung (40) eine erste Schicht (44), die eine erste Öffnung (46) definiert, und eine zweite Schicht (48), die eine zweite Öffnung (50) definiert, einschließt, wobei die erste Öffnung (46) und die zweite Öffnung (50) ausgerichtet sind und die erste Schicht (44) und die zweite Schicht (48) an der ersten Öffnung (46) und der zweiten Öffnung (50) aneinander befestigt sind, und wobei die erste Schicht (44) und die zweite Schicht (48) jeweils einen Umfang definieren, an dem sie jeweils an einer der inneren Oberfläche oder der äußeren Oberfläche der röhrenförmigen Graftkomponente (12) befestigt sind.

3. Stentgraft nach Anspruch 2, wobei der Ring (36) eine Vielzahl von strahlenundurchlässigen Markierungsbändern einschließt.

4. Stentgraft nach Anspruch 3, wobei mindestens ein Teil der strahlenundurchlässigen Markierungsbänder durch Gewebe (60) getrennt von der Auskleidung (40) eingekapselt ist, und optional das Gewebe (60) getrennt von der Auskleidung (40), das die strahlenundurchlässigen Markierungen einkapselt, an der röhrenförmigen Graftkomponente (12) fixiert ist.

5. Stentgraft nach Anspruch 3, wobei die strahlenundurchlässigen Markierungsbänder an die röhrenförmige Graftkomponente (12) genäht sind.

6. Stentgraft nach Anspruch 3, wobei mindestens ein Teil der strahlenundurchlässigen Markierungen (58) als mindestens eines von länglichen Zylindern und Röhren geformt ist.

7. Stentgraft nach Anspruch 3, ferner einschließend einen Tragring (62), der sich um die Fenestration herum erstreckt und optional eines oder mehrere von: selbstexpandierend ist, eine Formgedächtnislegierung einschließt und durch die Auskleidung (40) abgedichtet ist.

8. Stentgraft nach Anspruch 7, wobei sich der Tragring (62) um den Umfang der ersten Schicht oder der zweiten Schicht herum erstreckt.

9. Stentgraft nach Anspruch 7, wobei der Tragring (62) an die röhrenförmige Graftkomponente (12) genäht ist.

10. Stentgraft nach Anspruch 1, wobei der Ring (36) ein durchgehender Tragring ist.

11. Stentgraft nach Anspruch 10, ferner einschließend mindestens eine strahlenundurchlässige Markierung (68, 70, 72) zwischen der röhrenförmigen Graftkomponente (12) und der Auskleidung (40).

12. Stentgraft nach Anspruch 11, wobei sich die mindestens eine strahlenundurchlässige Markierung (68, 70) um die Fenestration (24) der röhrenförmigen Graftkomponente (12) herum oder zwischen dem durchgehenden Tragring (36) und der Fenestration (24) der röhrenförmigen Graftkomponente (12) erstreckt.

13. Stentgraft nach Anspruch 12, wobei sich die mindestens eine strahlenundurchlässige Markierung (72) um den durchgehenden Tragring herum erstreckt, wobei sich der durchgehende Tragring (36) zwischen der strahlenundurchlässigen Markierung und der Fenestration (24) der röhrenförmigen Graftkomponente (12) befindet.

14. Stentgraft nach Anspruch 1, wobei die röhrenförmige Graftkomponente (12) distal zu der Fenestration (24) gegabelt ist.

## Revendications

1. Endoprothèse couverte, comprenant :
a) un composant de corps tubulaire (12) définissant une surface intérieure (14), une surface extérieure (16), une extrémité proximale ouverte (18), une extrémité distale ouverte et au moins une fenestration (24) ;
b) une endoprothèse proximale au niveau du composant de corps tubulaire en position proximale par rapport à l'au moins une fenestration ;
c) un anneau (36) au niveau du composant de corps tubulaire (12) et s'étendant le long du composant de corps tubulaire (12) et autour de l'au moins une fenestration (24) ; et **caractérisée par :**
d) un revêtement (40) s'étendant entre la surface intérieure (14) et la surface extérieure (16) et à travers la fenestration (24), l'anneau étant ainsi protégé de toute exposition au niveau d'une surface (16) du composant de corps tubulaire par le revêtement (40) et au niveau de l'autre surface du composant de corps tubulaire par le composant de tubulaire (12) et le revêtement (40) ; et **en ce que :**
l'anneau (36) s'étend le long du composant de corps tubulaire (12) à l'extérieur d'un périmètre (38) qui définit la fenestration (24), moyennant quoi une partie du composant de corps tubulaire (12) s'étend depuis l'anneau (36) jusqu'au périmètre (38) de la fenestration (24) définie par le composant de corps tubulaire (12), de telle sorte que le diamètre de la fenestration (24), définie par le composant de corps tubulaire (12), est plus petit que le diamètre interne de l'anneau (36).

2. Endoprothèse couverte selon la revendication 1, dans laquelle le revêtement (40) comporte une première couche (44) définissant une première ouverture (46) et une seconde couche (48) définissant une seconde ouverture (50), dans laquelle la première ouverture (46) et la seconde ouverture (50) sont alignées et la première couche (44) et la seconde couche (48) sont fixées l'une à l'autre au niveau de la première ouverture (46) et de la seconde ouverture (50), et dans laquelle la première couche (44) et la seconde couche (48) définissent chacune un périmètre au niveau duquel elles sont chacune fixées à l'une parmi la surface intérieure ou la surface extérieure du composant de corps tubulaire (12).

3. Endoprothèse couverte selon la revendication 2, dans laquelle l'anneau (36) comporte une pluralité de bandes de marqueurs radio-opaques.

4. Endoprothèse couverte selon la revendication 3, dans laquelle au moins une partie des bandes de marqueurs radio-opaques sont encapsulées par un tissu (60) séparé du revêtement (40), et éventuellement le tissu (60) séparé du revêtement (40) qui encapsule les marqueurs radio-opaques est fixé au composant de corps tubulaire (12).

5. Endoprothèse couverte selon la revendication 3, dans laquelle les bandes de marqueurs radio-opaques sont cousues au composant de corps tubulaire (12).

6. Endoprothèse couverte selon la revendication 3, dans laquelle au moins une partie des marqueurs radio-opaques (58) présentent la forme d'au moins l'un parmi cylindres et tubes allongés.

7. Endoprothèse couverte selon la revendication 3, comportant en outre un anneau de support (62) s'étendant autour de la fenestration et éventuellement une ou plusieurs caractéristiques parmi : être autodéployable, comporter un alliage à mémoire de forme et être protégé par le revêtement (40).

8. Endoprothèse couverte selon la revendication 7, dans laquelle l'anneau de support (62) s'étend autour du périmètre de la première couche ou de la seconde couche.

9. Endoprothèse couverte selon la revendication 7, dans laquelle l'anneau de support (62) est cousu au composant de corps tubulaire (12).

10. Endoprothèse couverte selon la revendication 1, dans laquelle l'anneau (36) est un anneau de support continu.

11. Endoprothèse couverte selon la revendication 10, comportant en outre au moins un marqueur radio-opaque (68, 70, 72) entre le composant de corps tubulaire (12) et le revêtement (40).

12. Endoprothèse couverte selon la revendication 11, dans laquelle l'au moins un marqueur radio-opaque (68, 70) s'étend autour de la fenestration (24) du composant de corps tubulaire (12) ou entre l'anneau de support continu (36) et la fenestration (24) du composant de corps tubulaire (12).

13. Endoprothèse couverte selon la revendication 12, dans laquelle l'au moins un marqueur radio-opaque (72) s'étend autour de l'anneau de support continu, moyennant quoi l'anneau de support continu (36) est situé entre le marqueur radio-opaque et la fenestration (24) du composant de corps tubulaire (12).

14. Endoprothèse couverte selon la revendication 1, dans laquelle le composant de corps tubulaire (12) est bifurqué distalement par rapport à la fenestration (24).
